(19) Europäisches Patentamt
European Patent Office
Office européen des brevets

(11) Publication number: **0 141 419**
**B1**

(12) **EUROPEAN PATENT SPECIFICATION**

(45) Date of publication of patent specification: **09.03.88**

(21) Application number: **84113353.1**

(22) Date of filing: **06.11.84**

(51) Int. Cl.⁴: **C 07 C 59/52,** C 07 C 62/32, C 07 C 69/732, C 07 C 103/58, C 07 C 103/60 // C09K15/24, C08K5/09, C08K5/10, C08K5/20

(54) **3,5-Dialkyl-4-hydroxyphenyl-substituted derivatives.**

(30) Priority: **07.11.83 US 549033**
**07.11.83 US 549034**
**07.11.83 US 549035**

(43) Date of publication of application:
**15.05.85 Bulletin 85/20**

(45) Publication of the grant of the patent:
**09.03.88 Bulletin 88/10**

(84) Designated Contracting States:
**CH DE FR GB LI NL**

(56) References cited:
**GB-A-1 521 567**
**US-A-3 282 939**
**US-A-3 455 994**
**US-A-4 390 723**

(73) Proprietor: **The B.F. GOODRICH Company**
**Dept. 0015 WHB-6 500 South Main Street**
**Akron, Ohio 44318 (US)**

(72) Inventor: **Lai, John Ta-Yuan**
**3465 Ridge Park Blvd.**
**Broadview Heights Ohio 4414 (US)**

(74) Representative: **von Kreisler, Alek, Dipl.-Chem. et al**
**Deichmannhaus am Hauptbahnhof**
**D-5000 Köln 1 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 141 419 B1**

**Description**

3,5 Dialkyl-4-hydroxyphenyl substituted alkyl ester derivatives having similar substituents have been known from US—PA 3 455 994. 3,5 Dialkyl-4-hydroxyphenyl substituted acid and amide derivatives respectively having ressembling substitutents, have been disclosed in US—PS 4 390 723 and US—PS 3 282 939 respectively.

This invention relates to novel 3,5-dialkyl-4-hydroxyphenyl-substituted derivatives. The derivatives include substituted amides, acetic acids and acetic acid esters. The 3,5-dialkyl-4-hydroxyphenyl-substituted amides are prepared by a new process by reacting a 2,6-di-alkylphenol with an aliphatic, cycloaliphatic or alkaryl ketone; a haloform; and an amine in the presence of alkali metal hydroxide. The substituted acetic acids of this invention are prepared by a new process by reacting a 2,6-di-alkyl phenol with an appropriate ketone and a haloform in the presence of an alkali metal hydroxide. The substituted-acetic acid esters are prepared from 3,5-dialkyl-4-hydroxyphenyl-substituted acetic acids by reaction thereof with alcohols and alkyl halides. The derivatives are useful stabilizers for organic materials subject to attack and degradation by heat, oxygen and light, and form useful combinations for this purpose with other hindered phenol stabilizers. The compounds of this invention may be represented by general formula:

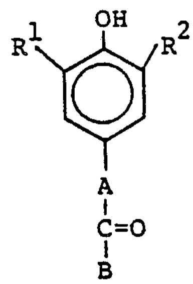

wherein

$R^1$ and $R^2$ are alkyl groups containing 1 to 12 carbon atoms, cycloalkyl groups containing 5 to 12 carbon atoms, or alkylcycloalkyl, aryl or alkaryl groups wherein the alkyl groups thereon contain 1 to 8 carbon atoms and the aryl groups are phenyl or naphthyl;

A is (1) an alkyl group of the formula

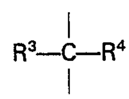

wherein $R^3$ and $R^4$ are alkyl groups containing 1 to 18 carbon atoms, or (2) a cycloalkyl group or alkaryl group defined as above;

B is OH, the formula

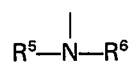

wherein $R^5$ is alkyl, aryl, cycloalkyl or alkaryl defined as above or an alkylidene group containing 1 to 18 carbon atoms,

$R^5$ and $R^6$ are hydrogen, alkyl, hydroxyalkyl, aminoalkyl, aryl, cycloalkyl or arylalkyl defined as above or the formula —O—$R^7$, where $R^7$ is an alkylidene, cycloalkyl or alkaryl radical defined as above.

DETAILED DESCRIPTION

The novel 3,5-dialkyl-4-hydroxyphenyl-substituted derivatives of this invention are represented by the general formula:

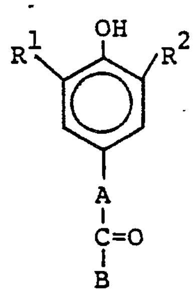

wherein

$R^1$ and $R^2$ are alkyl groups containing 1 to 12 carbon atoms, cycloalkyl groups containing 5 to 12 carbon

atoms, or alkylcycloalkyl, aryl or alkaryl groups wherein the alkyl groups thereon contain 1 to 8 carbon atoms and the aryl groups are phenyl or naphthyl;

A is (1) an alkyl group of the formula

$$R^3-\overset{|}{\underset{|}{C}}-R^4$$

wherein $R^3$ and $R^4$ are alkyl groups containing 1 to 18 carbon atoms, or (2) a cycloalkyl group or alkaryl group defined as above;

B is OH, the formula

$$R^5-\overset{|}{N}-R^6$$

wherein $R^5$ is alkyl, aryl, cycloalkyl or alkaryl defined as above or an alkylidene group containing 1 to 18 carbon atoms,

$R^5$ and $R^6$ are hydrogen, alkyl, hydroxyalkyl, aminoalkyl, aryl, cycloalkyl or arylalkyl defined as above or the formula —O—$R^7$, where $R^7$ is an alkylidene, cycloalkyl or alkaryl radical defined as above, preferably

$R^1$ is an alkyl group which contains 1 to 8 carbon atoms, $R^2$ is an alkyl group which contain 1 to 5 carbon atoms, $R^3$ and $R^4$ are alkyl groups which contain 1 to 8 carbon atoms and (1) is an alkyl group containing 1 to 8 carbon atoms and (2) is cyclohexyl, methylcyclohexyl, cycloheptyl or dicyclohexyl.

More preferred are derivatives wherein at least one of $R^1$ and $R^2$ is t-butyl or t-amyl, and $R^3$ and $R^4$ are alkyl groups containing 1 to 4 carbon atoms.

The novel 3,5-dialkyl-4-hydroxyphenyl-substituted amides of this invention have the general formula

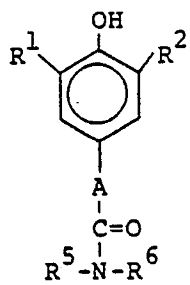

wherein

$R^1$ and $R^2$ are alkyl groups, cycloalkyl groups, alkylcycloalkyl, aryl or alkaryl groups defined as above; and

A is (1) an alkyl group having the formula

$$R^3-\overset{|}{\underset{|}{C}}-R^4$$

wherein

$R^3$ and $R^4$ are alkyl groups defined as above or (2) a cycloalkyl group or alkaryl groups defined as above, and $R^5$ is an alkyl, aryl, alkylidene, cycloalkyl or alkaryl radical as defined above, and

$R^5$ and $R^6$ are hydrogen, alkyl, hydroxyalkyl, aminoalkyl, cycloalkyl, aryl or alkaryl groups defined as above.

Preferably $R^1$ is an alkyl group containing 1 to 8 carbon atoms, and $R^2$ is an alkyl group containing 1 to 5 carbon atoms, and more preferably at least one of $R^1$ and $R^2$ is t-alkyl group including t-butyl and t-amyl; preferably $R^3$ and $R^4$ are alkyl groups containing 1 to 8 carbon atoms, and more preferably 1 to 4 carbon atoms, and (2) includes for example those groups derived from cyclohexanone, cycloheptanone, alkyl aryl ketones wherein the alkyl groups contain 1 to 8 carbon atoms and the aryl preferably is phenyl or naphthyl, and the like; $R^5$ and $R^6$ are hydrogen, alkyl, aryl, hydroxyalkyl, cycloalkyl and arylalkyl radicals containing from 1 to 18 carbon atoms, and at least one of $R^5$ or $R^6$ is an alkyl, aryl, hydroxyalkyl, aminoalkyl, alkoxyalkyl, cycloalkyl or alkaryl radical that preferably contains 1 to 8 carbon atoms.

These 3,5-dialkyl-4-hydroxyphenyl-substituted amides are prepared by a new process by reacting a 2,6,di-alkylphenol with an aliphatic, cycloaliphatic or alkaryl ketone, a haloform and an amine in the presence of alkali metal hydroxide. An organic solvent may be used, or large amounts of the ketone reactant may be employed. The amide is isolated in excellent yields by crystallizing the filtered reaction product.

Typical 2,6-dialkyl phenols used have the formula

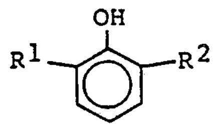

wherein $R^1$ and $R^2$ have the meanings set forth above, including 2-methyl-6-t-butylphenol, 2-ethyl-6-t-butylphenol, 2-propyl-6-t-butylphenol, 2-isopropyl-6-t-butylphenol, 2-n-butyl-6-t-butylphenol, 2,6-di-t-butylphenol, 2-n-amyl-6-t-butylphenol, 2-isoamyl-6-t-butylphenol, 2-hexyl-6-t-butylphenol, 2-heptyl-6-t-butylphenol, 2-isooctyl-6-t-butylphenol, 2-isopropanol-6-methyl, 2-n-butyl-6-isopropyl, 2-isoamyl-6-ethyl, 2-isoamyl-6-methyl, 2-isooctyl-6-methyl, 2-isooctyl-6-ethyl, 2-isooctyl-6-n-propyl, 2-isooctyl-6-n-hexyl, and the like.

The ketones used include dialkyl ketones, cycloalkanones, alkylcycloalkanones, and alkaryl ketones. Typical ketones that may be used in the novel process to make the new 3,5-dialkyl-4-hydroxyphenyl-substituted acetic acids of the invention are alkyl ketones of the formula

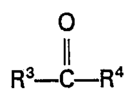

wherein $R^3$ and $R^4$ are alkyl radicals containing 1 to 18 carbon atoms, preferably 1 to 8 carbon atoms, $C_1$—$C_8$, including for example acetone, methyl ethyl ketone, methyl, n-propyl ketone, diethyl ketone, 2-hexanone, 3-hexanone, di-n-propyl ketone, 2-octanone, methyl isopropyl ketone, and the like. Also useful are the cycloalkane ketones containing 5 to 12 carbon atoms atoms such as cyclobutanone, cyclopentanone, cyclohexanone, cycloheptanone, cyclooctanone, cyclodecanone, methylcyclopentanone, methylcyclohexanone, dicyclohexyl ketone, alkaryl ketones when the alkyl group contains 1 to 4 carbon atoms such as acetophenone, o-methoxyacetophenone, p-chloroacetophenone, and the like; in amounts from at least about 1 mole of ketone per mole of 2,6-dialkylphenol, up to amounts sufficient for the ketones to be the solvent of the reaction, 10 moles or more. No more than about two moles is preferred when the ketone is a reactant only. Use of less than 1 mole reduces the product yield, and more than about 2 moles is unnecessary unless the ketone is the solvent.

The haloform, such as chloroform, is also used in a molar ratio of at least about one mole per mole of 2,6-dialkylphenol used, but preferably a slight molar excess is used up to about a 50 weight percent molar excess, i.e., 1.5 mole per mole of 2,6-dialkylphenol. While larger amounts may be used, there is no advantage realized, and lesser amounts will decrease the ultimate yield of the desired product. Bromoform may be substituted for the chloroform and excellent results will be obtained.

The alkali metal hydroxide is used in powder form, or in solution, and includes sodium hydroxide and potassium hydroxide. Preferably, a molar excess of the alkali metal hydroxide is used in relation to the amount of 2,6-dialkylphenol present. Normally from about 4 moles of alkali metal hydroxide per mole of 2,6-dialkylphenol up to 10 or more moles may be used, but the amount used preferably is about 4 to about 8 moles of hydroxide per mole of 2,6-dialkylphenol. Use of less than about 4 moles will reduce the yield of desired product.

The amines used in the practice of the invention include primary and, preferably, secondary amines. Better yields are generally obtained with secondary amines as compared to the primary amines. The amines include alkyl, cycloalkyl, and phenylalkyl amines. Typical amines include alkyl amines of the formula $RNH_2$ and $R_2NH$ wherein R is an alkyl or hydroxyalkyl radical containing 1 to 18 carbon atoms, a cycloalkyl radical containing 5 to 12 carbon atoms, and an arylalkyl radical, preferably phenyl or naphthyl wherein the alkyl groups contain 1 to 8 carbon atoms. Typical amines include methylamine, ethylamine, n-propylamine, isopropylmine, hexylamine, t-butylamine, t-octylamine, t-dodecylamine, decylamine, dodecylamine, octadecylamine, dimethylamine, diethylamine, dipropylamine, diisopropylamine, di-n-butylamine, di-n-amyl amine, dihexylamine, dioctyl amine, diisoctylamine, mixed sec-amines as ethylmethylamine and propylbutylamine, cyclohexylamine, benzylamine, 2-amino-2-ethyl-1,3-propanediol, 2-amino-2-methyl-1-propanol, aniline, 2-ethylaniline, 2,6-dimethylaniline, 2-isopropylaniline, 2,6-diethylaniline, 1,2-diaminocyclohexane, and the like. The amount of amine used in the reaction is based on one mole of the 2,6-dialkylphenol. While the amount may vary from less than a stoichiometric amount, resulting in low product yield, to larger excesses that are not required and have to be separated from the reaction mixture, normally greater than 1 mole to about 5 moles are used. Preferably from about 1 to 4 moles of amine per mole of 2,6-dialkylphenol are used.

Although a catalyst is not absolutely essential, it is preferably used in the reaction. These catalysts are typically onium salts, that is, quaternary compounds of ions derived from Groups VA and VIA elements that generally have the formula $(R_4Y)^+X^-$ wherein R are monovalent hydrocarbon radicals including alkyl, aryl, alkaryl, cycloalkyl and like radicals, Y is phosphorous or nitrogen and X is a halide, hydrogen sulfate, or like ions. Benzyltriethylammonium chloride has been found to be useful in amount in the range of about 0.01 to

about 0.1 mole of benzyltriethylammonium chloride per mole of 2,6-dialkylphenol used. Larger amounts are not necessary and lower amounts may be used if the reaction rate obtained is satisfactory. Other catalysts useful in the process that may be used include tetraalkyl ammonium salts such as tetrabutyl ammonium bromide, tetrabutyl ammonium hydrogen sulfate, methyl trioctylammonium chloride, tetraalkyl phosponium salts such as tetrabutyl phosphonium bromide, cetyltributyl phosphonium bromide, and the like.

The solvent used may be any polar organic solvent, including an excess of the ketone used in place of an added solvent. Typical solvents that may be used include methylene chloride, tetrahydrofuran, diethyl ether, dibutyl ether, dimethyl sulfone, 1,4-dioxane, carbon tetrachloride, toluene, and the like. The amounts of solvent used will vary from about 5 moles to 100 moles per mole of 2,6-dialkylphenol used. As has been stated, the solvent may be eliminated if an excess of the reactant ketone is used. In this case, the amount of ketone used, based on the moles of 2,6-dialkylphenol used may be from about 5 to about 20, preferably about 7.5 to 15 moles per mole of 2,6-dialkylphenol.

While the reactants may be added in any order, it is preferred that the alkali metal hydroxide be added last, over a period of time to control the exothermic reaction and preferably maintain the reaction below 30°C, preferably below about 10°C. The reaction temperature may be varied from about 0°C to about 30°C, but preferably is conducted from about 0°C to 10°C. The reaction time normally will vary from about 5 to about 15 hours.

The 3,5-dialkyl-4-hydroxyphenyl-substituted amides are readily isolated from the reaction mixture by filtration and washing the filtrate with aqueous inorganic acid, including hydrochloric or sulfuric acid. The filtrate is dried with a desiccant such as anhydrous sodium sulfate, and heated to dryness. This product may be recrystallized if desired.

The novel 3,5-dialkyl-4-hydroxyphenyl-substituted acetic acids of this invention have the general formula

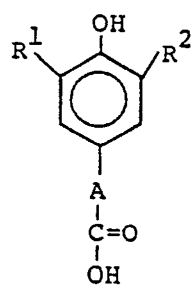

wherein $R^1$ and $R^2$ are alkyl groups, cycloalkyl groups, alkylcycloalkyl, aryl or alkaryl groups defined as above; and A is (1) an alkyl group having the formulas

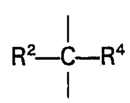

wherein $R^3$ and $R^4$ are alkyl groups defined as above or (2) a cycloalkyl group or alkaryl group defined as above. Preferably $R^1$ is an alkyl group containing 1 to 8 carbon atoms, and $R^2$ is an alkyl group containing 1 to 5 carbon atoms, and more preferably at least one of $R^1$ or $R^2$ is a t-alkyl group including including t-butyl and t-amyl; and A is (1) an alkyl group of the formula

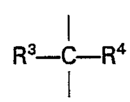

wherein $R^3$ and $R^4$ are preferably alkyl groups contaning 1 to 8 carbon atoms, and more preferably 1 to 4 carbon atoms; or (2) a cycloalkyl group containing 5 to 12 carbon atoms, including for example those derived from cyclohexanone, cycloheptanone, dicyclohexyl ketone, alkyl aryl ketones wherein the alkyl groups contain 1 to 8 carbon atoms and the aryl preferably is phenyl or naphthyl, and the like.

These 3,5-dialkyl-4-hydroxyphenyl-substituted acetic acids are prepared by a novel process by reacting a 2,6-di-alkylphenol with an aliphatic, cycloaliphatic or alkaryl ketone with a haloform in the presence of alkali metal hydroxide. An organic solvent may be used, or large amounts of the ketone reactant may be employed. The acid is isolated in excellent yields by treating the reaction product with an aqueous acid solution and crystallizing the reaction product.

Typical 2,6-dialkyl phenols used have the formula

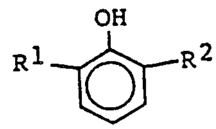

wherein $R^1$ and $R^2$ have the meanings set forth above, including 2-methyl-6-t-butylphenol, 2-ethyl-6-t-butylphenol, 2-propyl-6-t-butylphenol, 2-isopropyl-6-t-butylphenol, 2-n-butyl-6-t-butylphenol, 2,6-di-t-butylphenol, 2-n-amyl-6-t-butylphenol, 2-isoamyl-6-t-butylphenol, 2-hexyl-6-t-butylphenol, 2-heptyl-6-t-butylphenol, 2-isooctyl-6-t-butylphenol, 2-isopropyl-6-t-butylphenol, 2-n-butyl-6-isopropylphenol, 2-isoamyl-6-ethylphenol, 2-isoamyl-6-methylphenol, 2-isooctyl-6-methylphenol, 2-isooctyl-6-ethylphenol, 2-isooctyl-n-propphenol, 2-isooctyl-6-n-hexyolphenol, and the like.

The ketones used include dialkyl ketones, cycloalkanones, alkylcycloalkanones, and alkaryl ketones. Typical ketones that may be used in the novel process to make the new 3,5-dialkyl-4-hydroxyphenyl-substituted acetic acids of the invention are alkyl ketones of the formula

O
||
$R^3$—C—$R^4$

wherein $R^3$ and $R^4$ are alkyl radicals containing 1 to 18 carbon atoms, preferably 1 to 8 carbon atoms, $C_1$—$C_8$, including for example acetone, methyl ethyl ketone, methyl n-propyl ketone, diethyl ketone, 2-hexanone, 3-hexanone, di-n-propyl ketone, 2-octanone, methyl isobutyl ketone, and the like. Also useful are the cycloalkane ketones containing 5 to 12 carbon atoms such as cyclobutanone, cyclopentanone, cyclohexanone, cycloheptanone, cyclooctanone, 2-methylcyclopentanone, 2-methylcyclohexanone, dicyclohexyl ketone, alkaryl ketones when the alkyl group contains 1 to 4 carbon atoms such as acetophenone, o-methoxyacetophenone, p-chloroacetophenone, and the like; in amounts from at least about 1 mole of ketone per mole of 2,6-dialkylphenol, up to amounts sufficient for the ketones to be the solvent of the reaction, 10 moles or more. No more than about two moles is preferred when the ketone is a reactant only. Use of less than 1 mole reduces the product yield, and more than about 2 moles is unnecessary unless the ketone is the solvent.

The other essential reactants for the novel process of this invention include the alkali metal hydroxide in powder form, or in solution, including sodium hydroxide and potassium hydroxide. Preferably, a molar excess of the alkali metal hydroxide is used in relation to the amount of 2,6-dialkylphenol present. Normally from about 4 moles of alkali metal hydroxide per mole of 2,6-dialkylphenol up to 10 or more moles may be used, but the amount used preferably is about 4 to about 8 moles of hydroxide per mole of 2,6-dialkylphenol. Use of less than about 4 moles will reduce the yield of desired product.

The haloform, such as chloroform, is also used in a molar ratio of at least about one mole per mole of 2,6-dialkylphenol used, but preferably a slight molar excess is used up to about a 50 weight percent molar excess, i.e., 1.5 mole per mole of 2,6-dialkylphenol. While larger amounts may be used, there is no advantage realized, and lesser amounts will decrease the ultimate yield of the desired product. Bromoform may be substituted for the chloroform and excellent results will be obtained.

The 3,5-dialkyl-4-hydroxyphenyl-substituted acetic acids are readily isolated from the reaction mixture by adding sufficient aqueous inorganic acid, including hydrochloric or sulfuric acid, to the reaction mixture to isolate the acid, most of which is in the organic layer that forms. The aqueous layer is extracted with solvent to remove all traces of the acid, and this is added to the other organic layer and this mixture is dried with a desiccant such as anhydrous sodium sulfate, and heated to dryness. This product may be recrystallized if desired.

The 3,5-dialkyl-4-hydroxyphenyl-substituted acetic acid esters of this invention have the general formula

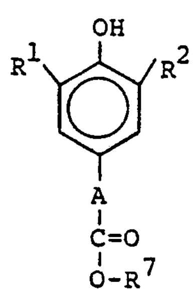

wherein $R^1$ and $R^2$ are alkyl groups, cycloalkyl groups, alkylcycloalkyl, aryl or alkaryl groups defined as above; and A is (1) an alkyl group having the formula

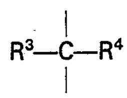

wherein $R^3$ and $R^4$ are alkyl groups defined as above or (2) a cycloalkyl group or alkaryl group defined as above, and $R^7$ is an alkylidene, cycloalkyl, or alkaryl radical defined as above.

Preferably $R^1$ is an alkyl group containing 1 to 8 carbon atoms, $C_1$—$C_8$ and $R^2$ is an alkyl group containing 1 to 5 carbon atoms, and more preferably at least one of $R^1$ or $R^2$ is a t-alkyl group including t-butyl and t-amyl; preferably $R^3$ and $R^4$ are alkyl groups containing 1 to 8 carbon atoms, $C_{1-18}$, and more preferably 1 to 4 carbon atoms, and (2) includes for example those groups derived from cyclohexanone, cycloheptanone, alkyl aryl ketones wherein the alkyl groups contain 1 to 8 carbon atoms and the aryl preferably is phenyl or naphthyl, and the like; $R^7$ may be derived from cycloalkyl radicals, arylalkyl radicals and the like, normally containing from 1 to about 18 carbon atoms. The esters are readily prepared from 3,5-dialkyl-4-hydroxyphenyl-substituted acetic acids by reaction thereof with alcohols, including polyols, and alkyl halides.

Typical alcohols include cycloalkanols, diols, triols and other polyols, cyclic, as well as alkaryl alcohols. The cycloalkanols include for example cyclopentanol, cyclohexanol, methyl cyclohexanol, and ethyl cycloheptanol. The arylalkyl alcohols include for example benzyl alcohol, phenylethanol, and the like. The polyols include diols such as ethylene glycol, propylene glycol, hexamethylene glycol, diethylene glycol, dipropylene glycol, other diols such as 1,4-butanediol and 2,3-butanediol; triols including glycerine, polyols such as erythritol, pentaerythritol; and the like.

The alkyl halides include mono- and di-halides having the general formula $R^5X$ or X—$R^5$—X wherein $R^5$ is an alkylene, cycloalkyl or alkaryl radical containing 1 to 18 carbon atoms, preferably 1 to 8 carbon atoms, and X is —I, —Cl, or —Br, preferably —I. Typical compounds include cyclohexyl iodide, benzyl iodide, α,α'-xylene dichloride, cyclohexyl iodide, cyclohexyl diiodide, cyclohexyl bromide, benzyl bromide, ethylene bromide, 1,3-propylene iodide, and the like.

The 3,5-dialkyl-4-hydroxyphenyl-substituted acetic acids used to prepare the esters are prepared by reacting a 2,6-di-alkylphenol with a cycloaliphatic or alkaryl ketone with a haloform in the presence of alkali metal hydroxide. An organic solvent may be used, or large amounts of the ketone reactant may be employed. The acid is isolated in excellent yields by treating the reaction product with an aqueous acid solution and crystallizing the reaction product.

Typical 2,6-dialkyl phenols used have the formula

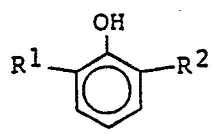

wherein $R^1$ and $R^2$ have the meanings set forth above, including 2-methyl-6-t-butylphenol, 2-ethyl-6-t-butylphenol, 2-propyl-6-t-butylphenol, 2-isopropyl-6-t-butylphenol, 2-n-butyl-6-t-butylphenol, 2,6-di-t-butylphenol, 2-n-amyl-6-t-butylphenol, 2-isoamyl-6-t-butylphenol, 2-hexyl-6-t-butylphenol, 2-heptyl-6-t-butylphenol, 2-isooctyl-6-t-butylphenol, 2-isopropyl-6-t-butyl, 2-n-butyl-6-isopropyl, 2-isoamyl-6-ethyl, 2-isoamyl-6-methyl, 2-isooctyl-6-methyl, 2-isooctyl-6-ethyl, 2-isooctyl-6-n-propyl, 2-isooctyl-6-n-hexyl, and the like.

The ketones used include cycloalkanones, alkylcycloalkanones, and alkaryl ketones. Typical ketones that may be used in the novel process to make the new 3,5-dialkyl-4-hydroxyphenyl-substituted acetic acids of the invention are ketones of the formula

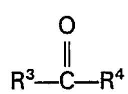

wherein $R^3$ and $R^4$ are cycloalkane ketones containing 5 to 12 carbon atoms such as cyclobutanone, cyclopentanone, cyclohexanone, cycloheptanone, cyclooctanone, methylcyclopentanone, methylcyclohexanone, dicyclohexyl ketone, alkaryl ketones when the alkyl group contains 1 to 4 carbon atoms such as acetophenone, o-methoxyphenone, p-chloroacetophenone, and the like; in amounts from at least about 1 mole of ketone per mole of 2,6-dialkylphenol, up to amounts sufficient for the ketones to be the solvent of the reaction, 10 moles or more.

The other reactants for the preparation of the acids include the alkali metal hydroxide in powder form, or in solution, including sodium hydroxide and potassium hydroxide. Preferably, a molar excess of the alkali metal hydroxide is used in relation to the amount of 2,6-dialkylphenol present. Normally from about 4 moles of alkali metal hydroxide per mole of 2,6-dialkyphenol up to 10 or more moles may be used, but the amount used preferably is about 4 to about 8 moles of hydroxide per mole of 2,6-di-alkylphenol. Use of less than about 4 moles will reduce the yield of desired product.

The haloform, such as chloroform, is also used in a molar ratio of at least about one mole per mole of 2,6-dialkylphenol used, but preferably a slight molar excess is used up to about a 50 weight percent molar excess, i.e., 1.5 mole per mole of 2,6-dialkylphenol. While larger amounts may be used, there is no advantage realized, and lesser amounts will decrease the ultimate yield of the desired product. Bromoform may be substituted for the chloroform and excellent results will be obtained.

The 3,5-dialkyl-4-hydroxyphenyl-substituted acetic acid esters are readily isolated from the reaction mixture by filtering and concentrating the filtrate to dryness. This product may be recrystallized if desired or the crude acid may be used to prepare the esters directly.

The practice of the invention is demonstrated in the following Examples. The structures of the 3,5-dialkyl-4-hydroxyphenyl-substituted derivatives were confirmed by infrared and nuclear magnetic resonance spectra. Molecular weights were determined and confirmed by field desorption mass spectra (FD/MS). Elemental analysis of carbon and hydrogen was done and the amounts found were consistent with the formulas of the materials. The practice of the invention is demonstrated in the following Examples.

### Example I

2-(3,5-Di-t-butyl-4-hydroxyphenyl)-2-methyl propanoic acid

0.1 mole of 2,6-di-t-butylphenol, 0.12 moles of chloroform, 0.15 mole of acetone and 0.003 mole of benzyltriethylammonium chloride were mixed with 100 ml of methylene chloride in a 500 ml three-neck reactor having a circulating cold bath. While stirring, 0.5 mole of powdered sodium hydroxide was added in small portions during a 30 minute period. After this addition, the reaction temperature was slowly raised to 10°C and stirred overnight. 200 ml of 4N HCl was then added slowly and the resulting 2 layers were separated. The aqueous layer was extracted twice with 50 ml of methylene chloride. The combined organic layers were dried with sodium sulfate and concentrated. The residue was washed with hexanes to obtain straw-colored crystals in >90% yield. The product may be recrystallized from aqueous ethanol to afford white solid. The melting point was 210—214°C; the molecular weight 292; carbon-calculated 73.93%, found 74.16%; hydrogen-calculated 9.65%, found 9.72%.

### Example II

2-(3,5-Di-t-butyl-4-hydroxyphenyl)-2-methylbutanoic acid

0.1 mole of 2,6-di-t-butylphenol, 0.12 mole of chloroform, 1 mole of 2-butanone, and 0.003 mole of benzyltriethylammonium were mixed in the 500 ml three-neck reactor while stirring. 0.5 mole of powdered sodium hydroxide was added in small portions during a 30 minute period. After the addition, the reaction temperature was slowly raised to 10°C and stirred overnight. 200 ml of 4N HCl then was added slowly. The resulting 2 layers were separated. The aqueous layer was extracted twice with 50 ml methylene chloride and this was combined with the organic layer, then dried with sodium sulfate and concentrated. The residue was washed with hexanes to obtain crystals in 80% yield. The melting point was 141—144°C; molecular weight 306; carbon-calculated 74.47%, found 74.60%; hydrogen-calculated 9.87%, found 9.89%.

### Example III

Δ-(3,5-Di-t-butyl-4-hydroxyphenyl)cyclohexanecarboxylic acid

0.1 mole of 2,6-di-t-butylphenol, 0.12 mole of chloroform, 1.0 mole of cyclohexanone, and 0.003 mole of benzyltriethylammonium chloride were mixed in the 500 ml three-neck reactor while stirring. 0.5 mole of powdered sodium hydroxide was added in small portions during a 30 minute period. After the addition, the reaction temperature was slowly raised to 10°C and stirred overnight. 200 ml of 4N HCl then was added slowly. The resulting 2 layers were separated. The aqueous layer was extracted twice with 50 ml methylene chloride and this was combined with the organic layer, dried with sodium sulfate and concentrated. The residue was washed with hexane to obtain crystals in 80% yield. The melting point was 187-191°C; molecular weight 332; carbon-calculated 75.86%, found 73.35%; hydrogen-calculated 9.70%, found 9.59%.

### Example IV

2-(3-methyl-5-t-butyl-4-hydroxyphenyl)-2-propionic acid

0.1 mole of 2-methyl,5-t-butylphenol, 0.12 mole of chloroform, 0.15 mole of acetone, and 0.003 mole of benzyltriethylammonium chloride were mixed with 100 ml methylene chloride in a 500 ml three-neck reactor equipped with a circulating cold bath. With stirring, 0.5 mole of powdered sodium hydroxide was added in small portions during a 30 minute period. After the addition, the reaction temperature was slowly raised to 10°C and stirred overnight. 200 ml of 4N HCl was added slowly. The two layers were separated. The aqueous layer was extracted twice with 50 ml of methylene chloride and this extract was combined with the organic layer. This was dried with sodium sulfate and concentrated. The residue was washed with hexane to obtain crystals in low yield. The melting point was 160—163°C; molecular weight 250; carbon-calculated 71.97%, found 71.94%; hydrogen-calculated 8.86%, found 8.86%.

These 3,5-dialkyl-4-hydroxyphenyl-substituted acetic acids may be used as stabilizers to protect organic materials subject to attack and degradation by oxygen, heat and light, where water solubility is not a problem. In many applications there is no exposure of the stabilized material to water and 3,5-dialkyl-4-hydroxyphenyl-α,α'-dialkyl acetic acids, for example, may be used as excellent stabilizers. Such materials that may be so stabilized include polymers, especially polyolefins; cellulosic materials; natural rubbers,

synthetic unsaturated diolefin derived elastomers, the vinylidene polymers made from unsaturated polymerizable monomers having at least one terminal $CH_2<$ group, including styrene, vinyl chloride and the alkyl acrylates; polycarbonates, polyurethanes, nylon type materials, epoxy resins, waxes, synthetic and petroleum-derived lubricating oils and greases; animal oils such as, for example, fat, tallow, lard, codliver oil, sperm oil; vegetable oils such as castor, linseed, peanut, palm, cotton seed, and the like; fuel oil, diesel oil, gasoline and the like.

A particularly useful application of these 3,5-dialkyl-4-hydroxyphenyl-substituted acetic acids is to make esters therefrom that are also of utility as stabilizers for organic materials subject to degradation.

## Example V

1-(3-methyl-5-t-butyl-4-hydroxyphenyl)cyclohexanecarboxylic acid

0.2 mole of 2-methyl,5-t-butylphenol, 0.12 mole of chloroform, 0.15 mole of cyclohexanone, and 0.003 mole of benzyltriethylammonium chloride were mixed with 100 ml methylene chloride in a 500 ml three-neck reactor equipped with a circulating cold bath. With stirring, 0.5 mole of powdered sodium hydroxide was added in small portions during a 30 minute period. After the addition, the reaction temperature was slowly raised to 10°C and stirred overnight. 200 ml of 4N HCl was added slowly. The two layers were separated. The aqueous layer was extracted twice with 50 ml of methylene chloride and this extract was combined with the organic layer. This was dried with sodium sulfate and concentrated. The residue was washed with hexane to obtain crystals in >80% yield.

## Example VI

P-xylene-α,α'-bis-[2-(3-t-butyl-5-methyl-4-hydroxyphenyl)-2-methyl-propionate]

0.02 mole of 2-(3-t-butyl-5-methyl-4-hydroxyphenyl)-propionic acid, 0.01 mole of α,α'-dichlorooxylene, 20 ml of dimethylformamide and 0.04 mole of potassium carbonate were mixed together in a reactor and stirred at 60°C temperature for about 4 hours. The reaction mixture was filtered and the filtrate was evaporated to dryness to obtain a white powder. The p-xylene-α,α'-bis-[2-(3-t-butyl-5-methyl-4-hydroxyphenyl)-2-methyl-propionate had a melting point of 188—192°C and a molecular weight of 602. Carbon, calculated was 75.71%, found 76.02%. Hydrogen, calculated was 8.36%, found 8.14%.

## Example VII

P-xylene-α,α'-bis-[2-(3,5-di-t-butyl-4-hydroxyphenyl)-2-methyl propionate]

0.02 mole of 2-(3,5-di-t-butyl-4-hydroxyphenyl)-2-methyl propionic acid, 0.01 mole of α,α'-dichloroxylene, 20 ml of dimethylformamide and 0.04 mole of potassium carbonate were mixed together in a reactor and stirred at 60°C temperature for about 4 hours. The reaction mixture was filtered and the filtrate was evaporated to dryness to obtain a white powder. The p-xylene-α,α'-bis-[2-(3,5-di-t-butyl-4-hydroxyphenyl)-2-methyl propionate] had a melting point of 180—184°C and a molecular weight of 687. Carbon, calculated was 76,93% found 76.58%. Hydrogen, calculated was 9.10% found 9.23%.

To demonstrate the stabilizing activity of the esters of this invention, test samples of the 3,5-dialkyl-4-hydroxyphenyl-substituted acid esters in polypropylene were prepared by mixing the stabilizer compounds with polypropylene in a Brabender Plasticorder fitted with a Cam-Head (mixing chamber). The polypropylene is first masticated for 1½ minutes at 190°C. Then the stabilizer is added, followed by 3 minutes additional mixing. The mass is removed and pressed into 20 mil thick sheets. From these sheets are cut 1" × 1" plaques for oven aging. Type C (3" × ⅛") tensil bars are cut for UV stability tests).

Thermal/oxidative stability (oven aging) testing consisted of aging the samples in triplicate in an air-circulating oven at 125°C. The time to catastrophic crumbling (failure) of the plaque was measured and reported as days to failure. Each sample contained 0.1 weight part of 3,5-dialkyl-4-hydroxyphenyl-substituted acid ester per 100 weight parts of polypropylene. The following results, in days to failure, were obtained:

| | 125°C |
|---|---|
| Control | 2 |
| P-xylene-α-bis[2-(3,5-di-t-butyl-4-hydroxyphenyl)-2-methyl propionate] | 12⅔ |
| P-xylene-α-bis[2-(3-t-butyl-5-methyl-4-hydroxyphenyl)-2-methyl propionate] | 10⅓ |

Additional samples of esters in polypropylene were prepared as described with 0.05 weight part of the novel esters listed below and 0.05 weight part of the commercial stabilizer distearyl thiodipropionate. For comparison purposes, the days to failure of polypropylene samples containing 0.1 weight part of the distearyl thiodipropionate was 12 days. The results obtained were:

| | 125°C |
|---|---|
| P-xylene-α,α'-bis[2-(3,5-di-t-butyl-4-hydroxyphenyl)-2-methyl propionate | 51 |
| P-xylene-α,α'-bis[2-(3-t-butyl-5-methyl-4-hydroxyphenyl)-2-methyl propionate | 60 |

When samples were prepared with 0.05 weight part of 2,2',2''-tris[(3,5-di-t-butyl-4-hydroxyphenyl)-propionyloxy]ethyl isocyanurate instead of the distearyl thiodipropionate, the following results were obtained: 69 days, and 53 days.

Samples containing 0.1 weight part of the listed 3,5-dialkyl-4-hydroxy-substituted acetic acid esters were tested for ultraviolet light stability, i.e., resistance to degradation by UV radiation. The samples were tested in an Atlas Xenon Weatherometer, Model No. 65-WR, equipped with a 6500 watt Xenon burner tube in accordance with ASTM #D2565—79—A. The black panel temperature was 60°C. The samples were subjected to an 18 minute cycle every 2 hours. The time in hours to a 50% loss in tensile strength was determined. For comparison purposes a sample with no ester was tested. The following results, in hours, were obtained.

| | Hours |
|---|---|
| Blank (Control) | 220 |
| P-xylene-α,α'-bis[2-(3,5-di-t-butyl-4-hydroxyphenyl)-2-methyl propionate | 500 |
| P-xylene-α,α'-bis[2-(3-t-butyl-5-methyl-4-hydroxyphenyl)-2-methyl propionate | 500 |

The 3,5-dialkyl-4-hydroxyphenyl-substituted acetic acid esters as defined herein provide exceptional heat stability and resistance to ultraviolet degradation to polyolefin polymers. The 3,5-dialkyl-4-hydroxyphenyl-substituted acetic acid esters are especially useful for the stabilization of α-monoolefin homopolymers and copolymers, wherein the α-monoolefin contains 2 to about 8 carbon atoms. High and low-density polyethylene, isotactic and atactic polypropylene, polyisobutylene, and poly(4-methyl-1-pentene) have excellent resistance to heat and oxygen when stabilized with the combinations of the present invention. Ethylene-propylene copolymers and ethylene-propylene terpolymers, generally containing less than about 10 percent by weight of one or more monomers containing multiple unsaturation provided, for example, by 1,4-hexadiene, dimethyl-1, 4,9-decatriene, dicyclopentadiene, vinyl norborene, ethylidene norborene, and the like, also provide excellent aging using the combination.

Other organic materials which can be stabilized in accordance with the present invention include both natural and synthetic polymers. For example, the stabilizers are useful for the stabilization of cellulosic materials; natural rubber, halogenated rubber, conjugated diene polymers, as, for instance, polybutadiene, copolymers of butadiene with styrene, acrylonitrile, acrylic acid, alkyl acrylates or methacrylates, methyl vinyl ketone, vinyl pyridine, etc.; polyisoprene, polychloroprene, and the like; vinyl polymers such as poly(vinyl chloride), poly(vinylidene chloride), copolymers of vinyl chloride with vinylidene chloride, polyvinyl acetate, copolymers or vinyl halide with butadiene, styrene, vinyl esters, α,β-unsaturated ketones and aldehydes, and the like; homopolymers and copolymers of acrylic monomers such as acrylic acid, methacrylic acid, methyl acrylate, methyl methacrylate, ethyl acrylate, 3-ethylhexyl acrylate, acrylamide, methacrylamide, N-methylol-acrylamide, haloacrylates, acrylonitrile, methacrylonitrile, and the like; epihalohydrin polymers; polyether or polyol-derived polyurethanes; acetal homopolymers and copolymers; polycarbonates; polyesters such as those derived from maleic, fumaric, itaconic, or terephthalic anhydrides; for example, polyethylene terephthalate; polyamides such as those derived from the reaction of hexa-methylene-diamine with adipic or sebacic acid; epoxy resins such as those obtained from the condensation of epichlorohydrin with bisphenols; ring opened olefin polymers and the like. Polymer blends, that is, physical admixture of two or more polymers may also be stabilized in accordance with the present invention.

In addition to polymeric materials, the present compounds may stabilize a wide variety of other organic materials. Such compounds include: waxes, synthetic and petroleum-derived lubricating oils and greases; animal oils such as, for example, fat, tallow, lard, codliver oil, sperm oil; vegetable oils such as castor, linseed, peanut, palm, cotton seed, and the like; fuel oil, diesel oil, gasoline and the like.

The 3,5-dialkyl-4-hydroxyphenyl-substituted acetic acid esters form useful stabilizer compositions with other stabilizers including hindered or partially hindered phenols. Typical of such phenols are the hydroxyphenylalkyleneyl isocyanurates such as the symmetrical tris(3,5-di-t-alkyl-4-hydroxybenzyl) isocyanurates; tetrakis[methylene 3-(3',5'-dialkyl-4'-hydroxyphenyl)propanoate] methanes wherein the alkyl groups contain 1 to 8 carbon atoms, such as tetrakis[methylene-3-(3',5'-di-t-butyl-4'-hydroxyphenyl)propanoate]-methane; alkyl-3-(3',5'-di-t-butyl-4'-hydroxyphenyl)propionates wherein the alkyl groups contain 1 to 18 carbon atoms, such as octadecyl 3-(3',5'-di-t-butyl-4-hydroxyphenyl) propionate; 1,3,5-trimethyl-2,4,6-tris[3,5-dialkyl-4-hydroxybenzyl)benzene; 1,3,5-tris(3,5-di-t-butyl-4-hydroxy-hydrocinnamoylethyl)-s-triazine-2,4,6-(1H,3H,5H)-trione; 2,2'-alkylidene bis (4,6-dialkylphenol)-s wherein the alkyl group contains 1 to 8 carbon atoms, such as 2,2'-methylene bis(4,6-di-t-butylphenol), 2,2'-ethylidene bis(4,6-di-t-butylphenol), and 2,2'-methylene bis (4-methyl-6-t-butylphenol), and the like.

For example, the following combination provided excellent ageing in polypropylene as shown in the Weatherometer: 0.05 phr of p-xylene-α,α'-[2-(3,5-di-t-butyl-4-hydroxyphenyl)-2-methyl propionate], 0.05 phr of 1,1'-(1,2-ethandiyl)bis(3,3,5,5-tetramethyl piperazinone), and 0.05 phr of 2,2',2''-tris[3(3,5-di-t-butyl-4-hydroxyphenyl)propionyloxy] ethyl isocyanurate — 1,630 hours.

The compounds are readily incorporated into materials to be patented by dissolving or dispersing them with the materials, in liquids, dispersions, solutions, and solid forms. If the material is a solid, especially a polymeric solid such as rubber or a plastic, the compounds can be admixed using mixers such as Banburys, extruders, two-roll mills, and the like, following conventional techniques. One way to disperse the compounds in plastic materials is to dissolve or suspend the compounds in a solvent or diluent, mix the mixture with a plastic in powder or solution form, and then evaporate the solvent.

Compositions containing the novel combination of compounds can also contain other known compounding ingredients such as fillers like carbon black, silica, metal carbonates, talc, and the like; pigments and colorants; curative ingredients like sulfur and peroxides, and vulcanization accelerators; fungicides; processing aids, reinforcing agents and standard ingredients known to the art. Other ingredients known in the art as ultraviolet light, thermal and/or oxidative stabilizers can also be used in the stabilized compositions.

## Example VIII

N,N'-Diethyl-2-(3,5-di-t-butyl-4-hydroxyphenyl)-2-methyl-propionamide

0.1 mole of 2,6-di-t-butylphenol, 0.2 mole of acetone, 0.12 mole of chloroform, 0.2 mole of diethylamine, and 0.003 mole of benzyltriethylammonium chloride were added to 100 ml of methylene chloride in a reactor and mixed by stirring while being cooled by a circulating cold bath to 0°C. 0.5 mole of powdered sodium hydroxide was slowly added over a period of 30 minutes. The temperature was slowly raised to 10°C and the reaction mixture was stirred overnight. The reaction mix from the reactor was filtered. The solid residue was washed with methylene chloride and the wash added to the filtrate. The filtrate was washed with 200 ml of 4N HCl and was then dried over sodium sulfate. The filtrate was evaporated to dryness and the dried product was washed with hexane. The resulting amide was a white solid that had a melting point of 128—9°C and a molecular weight of 347. By elemental analysis it was determined that the amide contained 76.05% carbon (76.03% calculated), 10.40% hydrogen (calculated 10.73%) and 4.13% nitrogen (calculated 4.03%).

## Example IX

N,N'-Diethyl-2-(3,5-di-t-butyl-4-hydroxyphenyl)-2-methyl-butanamide

0.1 mole of 2,6-di-t-butylphenol, 0.2 mole of methyl ethyl ketone, 0.12 mole of chloroform, 0.2 mole of diethylamine, and 0.003 mole of benzyltriethylammonium chloride were added to 100 ml of methylene chloride in a reactor and mixed by stirring while being cooled by a circulating cold bath to 0°C. 0.5 mole of powdered sodium hydroxide was slowly added over a period of 30 minutes. The temperature was slowly raised to 10°C and the reaction mixture was stirred overnight. The reaction mix from the reactor was filtered. The solid residue was washed with methylene chloride and the wash added to the filtrate. The filtrate was washed with 200 ml of 4N HCl and was then dried over sodium sulfate. The filtrate was evaporated to dryness and the dried product was washed with hexane. The resulting amide was a white solid that had a melting point of 109—112°C and a molecular weight of 361. By elemental analysis it was determined that the amide contained 76.66% carbon (76.40% calculated), 10.98% hydrogen (calculated 10.87%) and 3.54% nitrogen (calculated 3.87%).

## Example X

N,N'-Diethyl-2-(3,5-di-t-butyl-4-hydroxyphenyl)-2,2-pentamethylene acetamide

0.1 mole of 2,6-di-t-butylphenol, 0.2 mole of cyclohexanone, 0.12 mole of chloroform, 0.2 mole of diethylamine, and 0.003 mole of benzyltriethylammonium chloride were added to 100 ml of methylene chloride in a reactor and mixed by stirring while being cooled by a circulating cold bath to 0°C. 0.5 mole of powdered sodium hydroxide was slowly added over a period of 30 minutes. The temperature was slowly raised to 10°C and the reaction mixture was stirred overnight. The reaction mix from the reactor was filtered. The solid residue was washed with methylene chloride and the wash added to the filtrate. The filtrate was washed with 200 ml of 4N HCl and was then dried over sodium sulfate. The filtrate was evaporated to dryness and the dried product was washed with hexane. The resulting amide was a white solid that had a melting point of 102—104°C and a molecular weight of 387. By elemental analysis it was determined that the amide contained 77.72% carbon (77.47% calculated), 10.83% hydrogen (calculated 10.66%) and 3.57% nitrogen (calculated 3.61%).

Example XI

N,N′-Dibutyl-2-(3-t-butyl-5-methyl-4-hydroxyphenyl)-2-methylpropionamide

0.1 mole of 2-t-butyl-6-methylphenol, 0.2 mole of acetone, 0.12 mole of chloroform, 0.2 mole of di-n-butylamine, and 0.003 mole of benzyltriethylammonium chloride were added to 100 ml of methylene chloride in a reactor and mixed by stirring while being cooled by a circulating cold bath to 0°C. 0.5 mole of powdered sodium hydroxide was slowly added over a period of 30 minutes. The temperature was slowly raised to 10°C and the reaction mixture was stirred overnight. The reaction mix from the reactor was filtered. The solid residue was washed with methylene chloride and the wash added to the filtrate. The filtrate was washed with 200 ml of 4N HCl and was then dried over sodium sulfate. The filtrate was evaporated to dryness and the dried product was washed with hexane. The resulting amide was a white solid that had a melting point of 115—117°C and a molecular weight of 361. By elemental analysis it was determined that the amide contained 76.67% carbon (77.40% calculated), 10.95% hydrogen (calculated 10.87%) and 3.79% nitrogen (calculated 3.87%).

Other amides were made following the general procedure of Examples VIII—XI as follows:

XII. N-t-butyl-2-methyl-2-(3,5-di-t-butyl-4-hydroxyphenyl)propionamide by reacting together 0.1 mole of 2,6-di-t-butylphenol, 1.0 mole of acetone, 0.15 mole of chloroform, 0.2 mole of t-butylamine, and 0.5 mole of sodium hydroxide.

XIII. N-(o-isopropylphenyl-2-(3,5-di-t-butyl-4-hydroxyphenyl)-2,2-pentamethylene acetamide by reacting together 0.1 mole of 2,6-di-t-butylphenol, 1.0 mole of cyclohexanone, 0.15 mole of chloroform, 0.1 mole of o-isopropylaniline and 0.5 mole of sodium hydroxide.

XIV. N-(2,6-diethylphenyl)-2-(3,5-di-t-butyl-4-hydroxyphenyl)-2-methylpropionamide, by reacting together 0.1 mole of 2,6-di-t-butylphenol, 1.0 mole of acetone, 0.15 mole of chloroform, 0.15 mole of 2,6-diethylaniline and 0.5 mole of sodium hydroxide.

XV. N-cyclohexyl-2-(3,5-di-t-butyl-4-hydroxyphenyl)-2-methylpropionamide by reacting together 0.1 mole of 2,6-di-t-butylphenol, 1.0 mole of acetone, 0.12 mole of chloroform, 0.1 mole of cyclohexylamine and 0.5 mole of sodium hydroxide.

XVI. N-(1-hydroxy-2-methyl-2-propyl)-2-(3,5-di-t-butyl-4-hydroxyphenyl)-2-methyl propionamide by reacting together 0.1 mole of 2,6-di-t-butylphenol, 1.0 mole of acetone, 0.13 mole of chloroform, 0.1 mole of 2-amino-2-methylpropanol, and 0.5 mole of sodium hydroxide.

XVII. N-methyl-N′-(2-hydroxyethyl)-2-(3,5-di-t-butyl-4-hydroxyphenyl)-2-methylpropionamide by reacting together 0.1 mole of 2,6-di-t-butylphenol, 1.0 mole of acetone, 0.15 mole of chloroform, 0.3 mole of N-methylethanolamine, and 0.5 mole of sodium hydroxide.

To demonstrate the stabilizing activity of the amides of this invention, test samples of the amides in polypropylene were prepared by mixing with polypropylene in a Brabender Plasticorder fitted with a Cam-Head (mixing chamber). The polypropylene is first masticated for 1½ minutes at 190°C. Then the stabilizer is added, followed by 3 minute additional mixing. The mass is removed and pressed into 20 mil thick sheets. From these sheets are cut 1″ × 1″ plaques for oven aging. Type C (3″ × ⅛″) tensil bars are cut for UV stability tests.

Thermal/oxidative stability (oven aging) testing consisted of aging the samples in triplicate in an air-circulating oven at 125°C. The time to catastrophic crumbling (failure) of the plaque was measured and reported as days to failure. Each sample contained 0.1 weight part of amide per 100 weight parts of polypropylene. The following results, in days to failure, were obtained:

| | 125°C |
|---|---|
| Control | 2 |
| I. N,N′-Dibutyl-2-(3,5-di-t-butyl-4-hydroxyphenyl)-2-methylpropionate | 5 |
| II. N,N′-Diethyl-2-(3,5-di-t-butyl-4-hydroxyphenyl)-2-methyl-butanamide | 2⅓ |
| III. N,N′-Diethyl-2-(3,5-di-t-butyl-4-hydroxyphenyl)-2,2-pentamethylene acetamide | 3⅓ |
| IV. N,N′-Dibutyl-2-(3-t-butyl-5-methyl-4-hydroxyphenyl)-2-methyl propionamide | 3½ |

Samples containing 0.1 weight part of the listed 3,5-dialkyl-4-hydroxyphenyl-substituted amide were tested for ultraviolet light stability, i.e., resistance to degradation by UV radication. The samples were tested in an Atlas Xenon Weatherometer, Model No. 65-WR, equipped with a 6500 watt Xenon burner tube in accordance with ASTM #D2565—79—A. The black panel temperature was 60°C. The samples were subjected to an 18-minute water cycle every 2 hours. The time in hours to a 50% loss in tensile strength was determined. For comparison purposes a samples with no amide was tested. The following results, in hours were obtained:

| | Hours |
|---|---|
| Blank (Control) | 220 |
| I. N,N′-Dibutyl-2-(3,5-di-t-butyl-4-hydroxyphenyl)-2-methylpropionate | 920 |
| II. N,N′-Diethyl-2-(3,5-di-t-butyl-4-hydroxyphenyl)-2-methyl-butanamide | 600 |
| III. N,N′-Diethyl-2-(3,5-di-t-butyl-4-hydroxyphenyl)-2,2-pentamethylene acetamide | 590 |
| IV. N,N′-Dibutyl-2-(3-t-butyl-5-methyl-4-hydroxyphenyl)-2-methyl propionamide | 470 |

The substituted-3,5-dialkyl-4-hydroxyphenyl amides as defined herein provide exceptional heat stability and resistance to ultraviolet degradation to polyolefin polymers. They are especially useful for the stabilization of α-monoolefin homopolymers and copolymers, wherein the α-monoolefin contains 2 to about 8 carbon atoms. High and low-density polyethylene, isotactic and atactic polypropylene, polyisobutylene, and poly(4-methyl-1-pentene) have excellent resistance to heat and oxygen when stabilized with the combinations of the present invention. Ethylene-propylene copolymers and ethylene-propylene terpolymers, generally containing less than about 10 percent by weight of one or more monomers containing multiple unsaturatation provided, for example, by 1,4-hexadiene, dimethyl-1, 4,9-decatriene, dicyclopentadiene, vinyl norborene, ethylidene norborene, and the like, also provide excellent aging using the combination.

Other organic materials which can be stabilized in accordance with the present invention include both natural and synthetic polymers. For example, the stabilizers are useful for the stabilization of cellulosic materials; natural rubber, halogenated rubber, conjugated diene polymers, as, for instance, polybutadiene, copolymers of butadiene with styrene, acrylonitrile, acrylic acid, alkyl acrylates or methacrylates, methyl vinyl ketone, vinyl pyridine, etc.; polyisoprene, polychloroprene, and the like; vinyl polymers such as poly(vinyl chloride), poly(vinylidene chloride), copolymers of vinyl chloride with vinylidene chloride, polyvinyl acetate, copolymers of vinyl halide with butadiene, styrene, viny esters, α,β-unsaturated ketones and aldehydes, and the like; homopolymers and copolymers of acrylic monomers such as acrylic acid, methacrylic acid, methyl acrylate, methyl methacrylate, ethyl acrylate, 3-ethylhexyl acrylate, acrylamide, methacrylamide, N-methylolacrylamide, haloacrylates, acrylonitrile, methacrylonitrile, and the like; epihalohydrin polymers; polyether or polyol-derived polyurethanes; acetal homopolymers and copolymers; polycarbonates; polyesters such as those derived from maleic, fumaric, itaconic, or terephthalic anhydrides; for example, polyethylene terephthalate; polyamides such as those derived from the reaction of hexa-methylene-diamine with adipic or sebacic acid; epoxy resins such as those obtained from the condensation of epichlorohydrin with bisphenols; ring opened olefin polymers and the like. Polymer blends, that is, physical admixture of two or more polymers may also be stabilized in accordance with the present invention. In addition to the polymeric materials, the present compounds may stabilize a wide variety of other organic materials. Such compounds include: waxes, synthetic and petroleum-derived lubricating oils and greases; animal oils such as, for example, fat, tallow, lard, codliver oil, sperm oil; vegetable oils such as castor, linseed, peanut, palm, cotton seed, and the like; fuel oil, diesel oil, gasoline and the like.

The (3,5-dialkyl-4-hydroxyphenyl)-substituted amides may be used with other stabilizers including hindered or partially hindered phenols, hindered amine light stabilizers, phosphites, and o-hydroxybenzophenones. Typical of such phenols are the hydroxyphenylalkyleneyl isocyanurates such as the symmetrical tris(3,5-di-t-alkyl-4-hydroxybenzyl) isocyanurates; tetrakis[methylene 3-(3',5'-dialkyl-4'-hydroxyphenyl)-propanoate] methanes wherein the alkyl groups contain 1 to 8 carbon atoms, such as tetrakis [methylene 3-(3',5'-di-t-butyl-4'-hydroxyphenyl)propanoate]methane; alkyl (3-3',5'-di-t-butyl-4'-hydroxyphenyl)-propionates wherein the alkyl groups contain 1 to 18 carbon atoms, such as octadecyl 3-(3',5'-di-t-butyl-4-hydroxyphenyl)propionate; 1,3,5-trimethyl-2,4,6-tris[3,5-dialkyl-4-hydroxybenzyl)benzene; 1,3,5-tris(3,5-di-t-butyl-4-hydroxy-hydrocinnamoylethyl)-s-triazine-2,4,6-(1H,3H,5H)-trione; 2,2'-alkylidene bis (4,6-dialkylphenol)-s wherein the alkyl group contains 1 to 8 carbon atoms, such as 2,2'methylene bis(4,6-di-t-butylphenol), 2,2'-ethylidene bis(4,6-di-t-butylphenol), and 2,2'-methylene bis (4-methyl-6-t-butylphenyl), and the like.

As an example of such a combination 0.05 phr of N,N'-diethyl-2-(3,5-di-t-butyl-4-hydroxyphenyl)-2-methyl-propionamide, 0.05 phr of 1,1'-(1,2-ethanediyl)bis([3,3,5,5-tetramethyl piperazinone) and 0.05 phr of 2,2',2''-tris[3(3,5-di-t-butyl-4-hydroxyphenyl)propionyloxy]ethylisocyanurate in polypropylene provided a composition that was still stable after more than 2,000 hours in the Weatherometer.

The compounds are readily incorporated into materials to be patented by dissolving or dispersing them with the materials, in liquids, dispersions, solutions, and solid forms. If the material is a solid, especially a polymeric solid such as rubber or a plastic, the compounds can be admixed using mixers such as Banburys, extruders, two-roll mills, and the like, following conventional techniques. One way to disperse the compounds in plastic materials is to dissolve or suspend the compounds in a solvent or diluent, mix the mixture with a plastic in powder or solution form, and then evaporate the solvent.

Compositions containing the novel combination of compounds can also contain other known compounding ingredients such as fillers like carbon black, silica, metal carbonates, talc, and the like; pigments and colorants; curative ingredients like sulfur and peroxides, and vulcanization accelerators; fungicides; processing aids, reinforcing agents and standard ingredients known to the art. Other ingredients known in the art as ultraviolet light, thermal and/or oxidative stabilizers can also be used in the stabilized compositions.

**Claims**

1. 3,5-dialkyl-4-hydroxyphenyl substituted derivatives having the formula

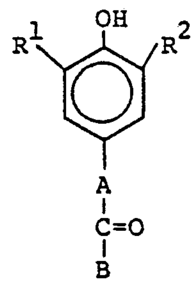

wherein

$R^1$ and $R^2$ are alkyl groups containing 1 to 12 carbon atoms, cycloalkyl groups containing 5 to 12 carbon atoms, or alkylcycloalkyl, aryl or alkaryl groups wherein the alkyl groups thereon contain 1 to 8 carbon atoms and the aryl groups are phenyl or naphthyl;

A is (1) an alkyl group of the formula

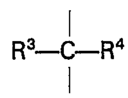

wherein $R^3$ and $R^4$ are alkyl groups containing 1 to 18 carbon atoms, or (2) a cycloalkyl group or alkaryl group defined as above;

B is OH, the formula

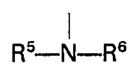

wherein $R^5$ is alkyl, aryl, cycloalkyl or alkaryl defined as above or an alkylidene group containing 1 to 18 carbon atoms,

$R^5$ and $R^6$ are hydrogen, alkyl, hydroxyalkyl, aminoalkyl, aryl, cycloalkyl or arylalkyl defined as above or the formula $—O—R^7$, where $R^7$ is an alkylidene, cycloalkyl or alkaryl radical defined as above.

2. 3,5-dialkyl-4-hydroxyphenyl-substituted derivatives of Claim 1 wherein $R^1$ is an alkyl group which contains 1 to 8 carbon atoms, $R^2$ is an alkyl group which contain 1 to 5 carbon atoms, $R^3$ and $R^4$ are alkyl groups which contain 1 to 8 carbon atoms and (1) is an alkyl group containing 1 to 8 carbon atoms and (2) is cyclohexyl, methylcyclohexyl, cycloheptyl or dicyclohexyl.

3. 3,5-dialkyl-4-hydroxyphenyl-substituted derivatives of Claim 2 wherein at least one of $R^1$ and $R^2$ is t-butyl or t-amyl, and $R^3$ and $R^4$ are alkyl groups containing 1 to 4 carbon atoms.

4. A composition comprising organic materials subject to degradation and stabilizing amount of 3,5-dialkyl-4-hydroxyphenyl-substituted derivatives of Claim 1.

5. 3,5-dialkyl-4-hydroxyphenyl-substituted acid derivatives of Claim 1 having the formula

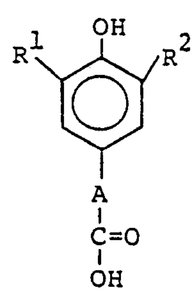

wherein $R^1$ and $R^2$ are alkyl groups, cycloalkyl groups, alkylcycloalkyl, aryl or alkaryl groups defined as in Claim 1; and A is (1) an alkyl group having the formulas

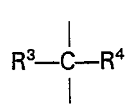

wherein $R^3$ and $R^4$ are alkyl groups defined as in Claim 1 or (2) a cycloalkyl group or alkaryl group defined as in Claim 1.

6. 3,5-dialkyl-4-hydroxyphenyl-substituted acids of Claim 5 wherein $R^1$ is an alkyl group which contains 1 to 8 carbon atoms, $R^2$ is an alkyl group which contains 1 to 5 carbon atoms, $R^3$ and $R^4$ are alkyl groups which contain 1 to 8 carbon atoms, (1) is an alkyl group containing 1 to 8 carbon atoms and (2) is cyclohexyl, methylcyclohexyl, cycloheptyl, or dicyclohexyl.

7. 3,5-dialkyl-4-hydroxyphenyl-α,α'-dialkylacetic acids of Claim 6 wherein at least one of $R^1$ and $R^2$ is t-butyl or t-amyl, and $R^3$ and $R^4$ are alkyl groups containing 1 to 4 carbon atoms.

8. A compound according to claim 7 selected from
2-(3,5-di-t-butyl-4-hydroxyphenyl)-2-methyl propionic acid,
2-(3,5-di-t-butyl-4-hydroxyphenyl)-2-methylbutanoic acid,
2-(3,5-di-t-butyl-4-hydroxyphenyl)-cyclohexanecarboxylic acid, and
2-(3-methyl-5-t-butyl-4-hydroxyphenyl)-2-propanoic acid.

9. 3,5-dialkyl-4-hydroxyphenyl-substituted ester derivatives of Claim 1 having the formula

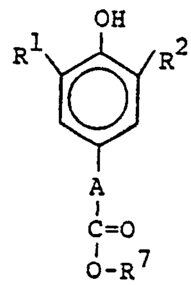

wherein $R^1$ and $R^2$ are alkyl groups, cycloalkyl groups, alkylcycloalkyl, aryl or alkaryl groups defined as in Claim 1; and A is (1) an alkyl group having the formula

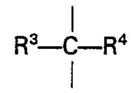

wherein $R^3$ and $R^4$ are alkyl groups defined as in Claim 1 or (2) a cycloalkyl group or alkaryl group defined as in Claim 1, and $R^7$ is an alkylidene, cycloalkyl, or alkaryl radical defined as in Claim 1.

10. 3,5-dialkyl-4-hydroxyphenyl-substituted acid esters of Claim 9 wherein $R^1$ is an alkyl group which contains 1 to 8 carbon atoms, $R^2$ is an alkyl group which contains 1 to 5 carbon atoms, $R^3$ and $R^4$ are alkyl groups which contain 1 to 8 carbon atoms, (1) is an alkyl group containing 1 to 8 carbon atoms, and (2) is cyclohexyl, methylcyclohexyl, cycloheptyl, or dicyclohexyl; and in $R^7$ the alkylidene group contains 1 to 8 carbon atoms, the cycloalkyl group contains 5 to 12 carbon atoms and the alkaryl group is a phenyl radical containing alkyl groups which have 1 to 3 carbon atoms.

11. 3,5-dialkyl-4-hydroxyphenyl-α,α'-dialkyl acetic acid esters of Claim 10 wherein at least one of $R^1$ and $R^2$ is t-butyl or t-amyl, and $R^3$ and $R^4$ are alkyl groups containing 1 to 4 carbon atoms.

12. A compound of claim 11 selected from
P-xylene-α,α'-bis[2-(3,5-di-t-butyl-4-hydroxyphenyl)-2-methyl propionate] and
P-xylene-α,α'-bis[2-(3-t-butyl-5-methyl-4-hydroxyphenyl)-2-methyl propionate].

13. 3,5-dialkyl-4-hydroxyphenyl-substituted amide derivatives of Claim 1 having the formula

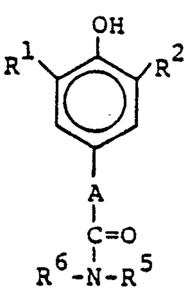

wherein $R^1$ and $R^2$ are alkyl groups, cycloalkyl groups, alkylcycloalkyl, aryl or alkaryl groups defined as in Claim 1; and A is (1) an alkyl group having the formula

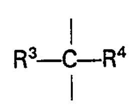

wherein $R^3$ and $R^4$ are alkyl groups defined as in Claim 1 or (2) a cycloalkyl groups or alkaryl groups defined as in Claim 1, and $R^5$ is an alkyl, aryl, alkylidene, cycloalkyl or alkaryl radical defined as in Claim 1, and $R^5$ and $R^6$ are hydrogen, alkyl, hydroxyalkyl, aminoalkyl, cycloalkyl, aryl or alkaryl groups defined as in Claim 1.

14. (3,5-dialkyl-4-hydroxyphenyl)-substituted amides of Claim 13 wherein $R^1$ is an alkyl group which contains 1 to 8 carbon atoms, $R^2$ is an alkyl group which contains 1 to 5 carbon atoms, and $R^3$ and $R^4$ are alkyl groups which contain 1 to 8 carbon atoms, (1) is an alkyl group containing 1 to 8 carbon atoms, and (2) is cyclohexyl, methylcyclohexyl, cycloheptyl, or dicyclohexyl; and in $R^5$ the alkyl or alkylidene group contains 1 to 8 carbon atoms, the cycloalkyl group contains 5 to 12 carbon atoms and the alkaryl is a phenyl radical containing alkyl groups which have 1 to 3 carbon atoms, and at least one of $R^5$ or $R^6$ is an alkyl, hydroxyalkyl, aminoalkyl, aryl, cycloalkyl or alkaryl group.

15. Substituted-2-(3,5-dialkyl-4-hydroxyphenyl amides of Claim 14 wherein at least one of $R^1$ and $R^2$ is t-butyl or t-amyl, $R^3$ and $R^4$ are alkyl groups containing 1 to 4 carbon atoms, and each of $R^5$ and $R^6$ containing 1 to 8 carbon atoms.

16. A compound of claim 15 selected from
N,N'-Dibutyl-2-(3,5-di-t-butyl-4-hydroxyphenyl)-2-methyl propionate,
N,N'-Diethyl-2-(3,5-di-t-butyl-4-hydroxyphenyl)-2-methyl butanamide,
N,N'-Diethyl-2-(3,5-di-t-butyl-4-hydroxyphenyl)-2,2-pentamethylene acetamide, and
N,N'-Dibutyl-2-(3-t-butyl-5-methyl-4-hydroxyphenyl)-2-methyl propionamide.

**Patentansprüche**

1. Substituierte 3,5-Dialkyl-4-hydroxyphenyl-Derivate der Formel

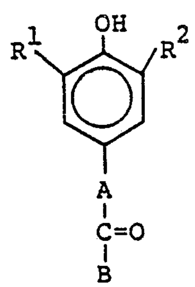

in der

$R^1$ und $R^2$ Alkyl-Gruppen mit 1 bis 12 Kohlenstoff-Atomen, Cycloalkyl-Gruppen mit 5 bis 12 Kohlenstoff-Atomen oder Alkylcycloalkyl-, Aryl- oder Alkaryl-Gruppen, worin die daran befindlichen Alkyl-Gruppen 1 bis 8 Kohlenstoff-Atome enthalten und die Aryl-Gruppen Phenyl oder Naphthyl sind, sind,

A (1) eine Alkyl-Gruppe der Formel

$$R^3—\overset{|}{\underset{|}{C}}—R^4,$$

worin $R^3$ und $R^4$ Alkyl-Gruppen mit 1 bis 18 Kohlenstoff-Atomen sind, oder (2) eine Cyloalkyl-Gruppe oder Alkaryl-Gruppe ist, wie sie im Vorstehenden definiert sind,

B OH, eine Gruppe der Formel

$$R^5—\overset{|}{N}—R^6,$$

worin $R^5$ Alkyl, Aryl, Cycloalkyl oder Alkaryl, wie sie im Vorstehenden definiert sind, oder eine Alkyliden-Gruppe mit 1 bis 18 Kohlenstoff-Atomen ist,

$R^5$ und $R^6$ Wasserstoff, Alkyl, Hydroxyalkyl, Aminoalkyl, Aryl, Cycloalkyl oder Arylalkyl, wie sie im Vorstehenden definiert sind, oder eine Gruppe der Formel —O—$R^7$ sind, worin $R^7$ ein Alkyliden-, cycloalkyl- oder Alkaryl-Rest ist, wie sie im Vorstehenden definiert sind.

2. Substituierte 3,5-Dialkyl-4-hydroxyphenyl-Derivate nach Anspruch 1, worin $R^1$ eine Alkyl-Gruppe mit 1 bis 8 Kohlenstoff-Atomen ist, $R^2$ eine Alkyl-Gruppe mit 1 bis 5 Kohlenstoff-Atomen ist, $R^3$ und $R^4$ Alkyl-Gruppen mit 1 bis 8 Kohlenstoff-Atomen sind und (1) eine Alkyl-Gruppe mit 1 bis 8 Kohlenstoff-Atomen ist und (2) Cyclohexyl, Methylcyclohexyl, Cycloheptyl oder Dicyclohexyl ist.

3. Substituierte 3,5-Dialkyl-4-hydroxyphenyl-Derivate nach Anspruch 2, worin wenigstens eine der Gruppen $R^1$ und $R^2$ t-Butyl oder t-Amyl ist und $R^3$ und $R^4$ Alkyl-Gruppen mit 1 bis 4 Kohlenstoff-Atomen sind.

4. Zusammensetzung, umfassend zersetzungsanfällige organische Stoffe und stabilisierende Mengen substituierter 3,5-Dialkyl-4-hydroxyphenyl-Derivate nach Anspruch 1.

5. Substituierte 3,5-Dialkyl-4-hydroxyphenyl-Säure-Derivate nach Anspruch 1 mit der Formel

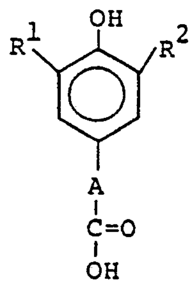

in der

$R^1$ und $R^2$ Alkyl-Gruppen, Cyloalkyl-Gruppen, Alkylcycloalkyl-, Aryl- oder Alkaryl-Gruppen sind, wie sie in Anspruch 1 definiert sind, und

A (1) eine Alkyl-Gruppe der Formel

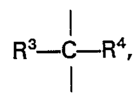

worin $R^3$ und $R^4$ Alkyl-Gruppen sind, wie sie in Anspruch 1 definiert sind, oder (2) eine Cyloalkyl-Gruppe oder Alkaryl-Gruppe ist, wie sie in Anspruch 1 definiert sind.

6. Substituierte 3,5-Dialkyl-4-hydroxyphenyl-Säuren nach Anspruch 5, worin $R^1$ eine Alkyl-Gruppe mit 1 bis 8 Kohlenstoff-Atomen ist, $R^2$ eine Alkyl-Gruppe mit 1 bis 5 Kohlenstoff-Atomen ist, $R^3$ und $R^4$ Alkyl-Gruppen mit 1 bis 8 Kohlenstoff-Atomen sind, (1) eine Alkyl-Gruppe mit 1 bis 8 Kohlenstoff-Atomen ist und (2) Cyclohexyl, Methylcyclohexyl, Cycloheptyl oder Dicyclohexyl ist.

7. 3,5-Dialkyl-4-hydroxyphenyl-α,α′-dialkyl-essigsäuren nach Anspruch 6, worin wenigstens eine der Gruppen $R^1$ und $R^2$ t-Butyl oder t-Amyl ist und $R^3$ und $R^4$ Alkyl-Gruppen mit 1 bis 4 Kohlenstoff-Atomen sind.

8. Verbindung nach Anspruch 7, ausgewählt aus
2-(3,5-Di-t-butyl-4-hydroxyphenyl)-2-methyl-propionsäure,
2-(3,5-Di-t-butyl-4-hydroxyphenyl)-2-methyl-buttersäure,
2-(3,5-Di-t-butyl-4-hydroxyphenyl)-cyclohexancarbonsäure und
2-(3-Methyl-5-t-butyl-4-hydroxyphenyl)-2-propansäure.

9. Substituierte 3,5-Dialkyl-4-hydroxyphenyl-Ester-Derivate nach Anspruch 1 mit der Formel

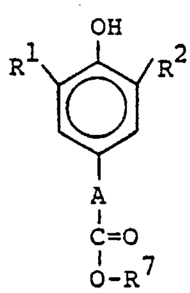

in der

$R^1$ und $R^2$ Alkyl-Gruppen, Cyloalkyl-Gruppen, Alkylcycloalkyl-, Aryl- oder Alkaryl-Gruppen sind, wie sie in Anspruch 1 definiert sind, und

A (1) eine Alkyl-Gruppe der Formel

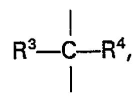

worin $R^3$ und $R^4$ Alkyl-Gruppen sind, wie sie in Anspruch 1 definiert sind, oder (2) eine Cyloalkyl-Gruppe oder Alkaryl-Gruppe ist, wie sie in Anspruch 1 definiert sind, und $R^7$ ein Alkyliden-, Cycloalkyl- oder Alkaryl-Rest ist, wie sie in Anspruch 1 definiert sind.

10. Substituierte 3,5-Dialkyl-4-hydroxyphenyl-Säureester nach Anspruch 9, worin $R^1$ eine Alkyl-Gruppe mit 1 bis 8 Kohlenstoff-Atomen ist, $R^2$ eine Alkyl-Gruppe mit 1 bis 5 Kohlenstoff-Atomen ist, $R^3$ und $R^4$ Alkyl-

Gruppen mit 1 bis 8 Kohlenstoff-Atomen sind, (1) eine Alkyl-Gruppe mit 1 bis 8 Kohlenstoff-Atomen ist und (2) Cyclohexyl, Methylcyclohexyl, Cycloheptyl oder Dicyclohexyl ist und in $R^7$ die Alkyliden-Gruppe 1 bis 8 Kohlenstoff-Atome enthält, die Cycloalkyl-Gruppe 5 bis 12 Kohlenstoff-Atome enthält und die Alkaryl-Gruppe ein Phenyl-Rest ist, der Alkyl-Gruppen mit 1 bis 3 Kohlenstoff-Atomen enthält.

11. 3,5-Dialkyl-4-hydroxyphenyl-α,α'-dialkyl-essigsäureester nach Anspruch 10, worin wenigstens eine der Gruppen $R^1$ und $R^2$ t-Butyl oder t-Amyl ist und $R^3$ und $R^4$ Alkyl-Gruppen mit 1 bis 4 Kohlenstoff-Atomen sind.

12. Verbindung nach Anspruch 11, ausgewählt aus p-Xylol-α,α'-bis[2-(3,5-di-t-butyl-4-hydroxyphenyl)-2-methylpropionat und p-Xylol-α,α'-bis[2-(3-t-butyl-5-methyl-4-hydroxyphenyl)-2-methylpropionat.

13. Substituierte 3,5-Dialkyl-4-hydroxyphenyl-Amid-Derivate nach Anspruch 1 mit der Formel

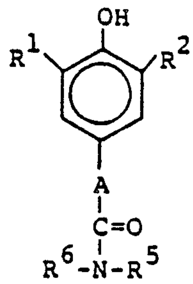

in der

$R^1$ und $R^2$ Alkyl-Gruppen, Cyloalkyl-Gruppen, Alkylcycloalkyl-, Aryl- oder Alkaryl-Gruppen sind, wie sie in Anspruch 1 definiert sind, und

A (1) eine Alkyl-Gruppe der Formel

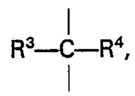

worin $R^3$ und $R^4$ Alkyl-Gruppen sind, wie sie in Anspruch 1 definiert sind, oder (2) eine Cyloalkyl-Gruppe oder Alkaryl-Gruppe ist, wie sie in Anspruch 1 definiert sind, und $R^5$ ein Alkyl-, Aryl-, Alkyliden-, Cycloalkyl- oder Alkaryl-Rest ist, wie sie in Anspruch 1 definiert sind, und $R^5$ und $R^6$ Wasserstoff, Alkyl-, Hydroxyalkyl-, Aminoalkyl-, Cycloalkyl-, Aryl- oder Alkaryl-Gruppen sind, wie sie in Anspruch 1 definiert sind.

14. Substituierte 3,5-Dialkyl-4-hydroxyphenyl-Amide nach Anspruch 13, worin $R^1$ eine Alkyl-Gruppe mit 1 bis 8 Kohlenstoff-Atomen ist, $R^2$ eine Alkyl-Gruppe mit 1 bis 5 Kohlenstoff-Atomen ist, $R^3$ und $R^4$ Alkyl-Gruppen mit 1 bis 8 Kohlenstoff-Atomen sind, (1) eine Alkyl-Gruppe mit 1 bis 8 Kohlenstoff-Atomen ist und (2) Cyclohexyl, Methylcyclohexyl, Cycloheptyl oder Dicyclohexyl ist und in $R^5$ die Alkyl- oder Alkyliden-Gruppe 1 bis 8 Kohlenstoff-Atome enthält, die Cycloalkyl-Gruppe 5 bis 12 Kohlenstoff-Atome enthält und die Alkaryl-Gruppe ein Phenyl-Rest ist, der Alkyl-Gruppen mit 1 bis 3 Kohlenstoff-Atomen enthält, und wenigstens eine der Gruppen $R^5$ und $R^6$ eine Alkyl-, Hydroxyalkyl-, Aminoalkyl-, Aryl-, Cycloalkyl- oder Alkaryl-Gruppe ist.

15. Substituierte 2-(3,5-Dialkyl-4-hydroxyphenyl-)amide nach Anspruch 14, worin wenigstens eine der Gruppen $R^1$ und $R^2$ t-Butyl oder t-Amyl ist und $R^3$ und $R^4$ Alkyl-Gruppen mit 1 bis 4 Kohlenstoff-Atomen sind und $R^5$ und $R^6$ jeweils 1 bis 8 Kohlenstoff-Atome enthalten.

16. Verbindung nach Anspruch 15, ausgewählt aus
N,N'-Dibutyl-2-(3,5-di-t-butyl-4-hydroxyphenyl)-2-methylpropionamid,
N,N'-Diethyl-2-(3,5-di-t-butyl-4-hydroxyphenyl)-2-methylbutanamid,
N,N'-Diethyl-2-(3,5-di-t-butyl-4-hydroxyphenyl)-2,2-pentamethylenacetamid und
N,N'-Dibutyl-2-(3-t-butyl-5-methyl-4-hydroxyphenyl)-2-methylpropionamid.

**Revendications**

1. Des dérivés substitués par un radical 3,5-dialkyl-4-hydroxyphényle répondant à la formule

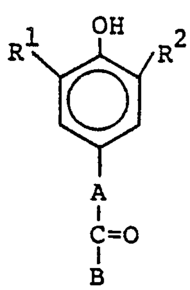

dans laquelle

$R^1$ et $R^2$ sont des groupes alkyles contenant 1 à 12 atomes de carbone, des groupes cycloalkyles contenant 5 à 12 atomes de carbone ou des groupes alkylcycloalkyles, aryles ou alcaryle dans lesquels les groupes alkyles renferment 1 à 8 atomes de carbone et les groupes aryle sont des phényle ou des naphtyle;

A est (1) un groupe alkyle de formule

|
$R^3$—C—$R^4$
|

dans laquelle $R^3$ et $R^4$ sont des groupes alkyles contenant 1 à 18 atomes de carbone ou (2) un groupe cycloalkyle ou alcaryle tel que défini précédemment;

B est OH, la formule

|
$R^5$—N—$R^6$

où $R^5$ est un alkyle, un aryle, un cycloalkyle ou un alcaryle tel que défini plus haut ou un groupe alkylidène contenant 1 à 18 atomes de carbone,

$R^5$ et $R^6$ sont des hydrogènes, des alkyles, des hydroxyalkyles, des aminoalkyles, des aryles, des cycloalkyles ou des arylalkyles tels que définis précédemment ou la formule —O—$R^7$ dans laquelle $R^7$ est un radical alkylidène, cycloalkyle ou alcaryle tel que défini précédemment.

2. Dérivés substitués par un radical 3,5-dialkyl-4-hydroxyphényle selon la revendication 1, dans lesquels $R^1$ est un groupe alkyle contenant 1 à 8 atomes de carbone, $R^2$ est un groupe alkyle contenant 1 à 5 atomes de carbone, $R^3$ et $R^4$ sont des groupes alkyles contenant 1 à 8 atomes de carbone, (1) est un groupe alkyle contenant 1 à 8 atomes de carbone et (2) est un groupe cyclohexyl, méthylcyclohexyle, cycloheptyle ou dicyclohexyle.

3. Dérivés substitués par un radical 3,5-dialkyl-4-hydroxyphényle selon la revendication 2, dans lesquels au moins un des $R^1$ et $R^2$ est un t-butyle ou un t-amyle et $R^3$ et $R^4$ sont des groupes alkyles contenant 1 à 4 atomes de carbone.

4. Une composition renfermant des matières organiques sujettes à la dégradation et une quantité stabilisante de dérivés substitués par un radical 3,5-dialkyl-4-hydroxyphényle selon la revendication 1.

5. Dérivés acides substitués par un radical 3,5-dialkyl-4-hydroxyphényle de la revendication 1, répondant à la formule

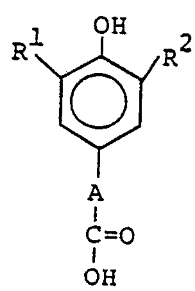

dans laquelle $R^1$ et $R^2$ sont des groupes alkyles, des groupes cycloalkyles, des groupes alkylcycloalkyles, aryle ou alcaryle tels que définis dans la revendication 1 et A est (1) un groupe alkyle de formule

|
$R^3$—C—$R^4$
|

où $R^3$ et $R^4$ sont des groupes alkyles tels que définis dans la revendication 1 ou (2) un groupe cycloalkyle ou alcaryle tel que défini dans la revendication 1.

6. Acides substitués par un radical 3,5-dialkyl-4-hydroxyphényle selon la revendication 5, dans lesquels $R^1$ est un groupe alkyle contenant 1 à 8 atomes de carbone, $R^2$ est un groupe alkyle contenant 1 à 5 atomes de carbone, $R^3$ et $R^4$ sont des groupes alkyles contenant 1 à 8 atomes de carbone, (1) est un groupe alkyle contenant 1 à 8 atomes de carbone et (2) est un cyclohexyle, un méthylcyclohexyle, un cycloheptyle ou un dicyclohexyle.

7. Acides 3,5-dialkyl-4-hydroxyphényl-α,α'-dialkylacétiques selon la revendication 6, dans lesquels au moins un des $R^1$ et $R^2$ est un t-butyle ou un t-amyle et $R^3$ et $R^4$ sont des groupes alkyles contenant 1 à 4 atomes de carbone.

8. Un composé selon la revendication 7 choisi parmi

l'acide 2-(3,5-di-t-butyl-4-hydroxyphényl)-2-méthylpropionique,

l'acide 2-(3,5-di-t-butyl-4-hydroxyphényl)-2-méthylbutanoïque,
l'acide 2-(3,5-di-t-butyl-4-hydroxyphényl)-cyclohexanecarboxylique, et
l'acide 2-(3-méthyl-5-t-butyl-4-hydroxyphényl)-6-propionïque.

9. Dérivés esters substitués par un radical 3,5-dialkyl-4-hydroxyphényle selon la revendication 1, répondant à la formule

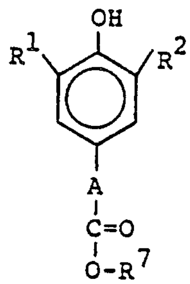

dans laquelle $R^1$ et $R^2$ sont des groupes alkyles, des groupes cycloalkyles, des groupes alkylcycloalkyles, aryle ou alcaryle, tels que définis dans la revendication 1, A est (1) un groupe alkyle de formule

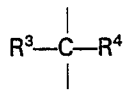

où $R^3$ et $R^4$ sont des groupes alkyles tels que définis dans la revendication 1 ou (2) un groupe cycloalkyle ou un groupe alcaryle tel que défini dans la revendication 1 et $R^7$ est un radical alkylidène, cycloalkyle ou alcaryle tel que défini dans la revendication 1.

10. Esters d'acides substitués par un radical 3,5-dialkyl-4-hydroxyphényle selon la revendication 9, dans lesquels $R^1$ est un groupe alkyle contenant 1 à 8 atomes de carbone, $R^2$ est un groupe alkyle contenant 1 à 5 atomes de carbone, $R^3$ et $R^4$ sont des groupes alkyles contenant 1 à 8 atomes de carbone, (1) est un groupe alkyle contenant 1 à 8 atomes de carbone et (2) est un groupe cyclohexyle, méthylcyclohexyle, cycloheptyle ou dicyclohexyle et, dans $R^7$, le groupe alkylidène contient 1 à 8 atomes de carbone, le groupe cycloalkyle contient 5 à 12 atomes de carbone et le groupe alcaryle est un radical phényle renfermant des groupes alkyles avec 1 à 3 atomes de carbone.

11. Esters d'acides 3,5-dialkyl-4-hydroxyphényl-α,α'-dialkylacétiques selon la revendication 10, dans lesquels au moins un des $R^1$ et $R^2$ est un t-butyle ou un t-amyle et $R^3$ et $R^4$ sont des groupes alkyles contenant 1 à 4 atomes de carbone.

12. Un composé selon la revendication 11 choisi parmi
le p-xylène-α,α'-bis[2-(3,5-di-t-butyl-4-hydroxyphényl)-2 méthylpropionate] et
le p-xylène-α,α'-bis[2-(3-t-butyl-5-méthyl-4-hydroxyphényl)-2-méthylpropionate].

13. Dérivés amides substitués par un radical 3,5-dialkyl-4-hydroxyphényle selon la revendication 1, répondant à la formule

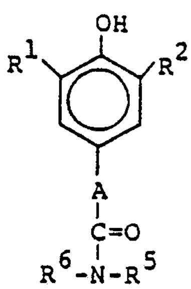

dans laquelle $R^1$ et $R^2$ sont des groupes alkyles, des groupes cycloalkyles, des groupes alkylcycloalkyles, aryle ou alcaryle telss que définis dans la revendication 1, A est (1) un groupe alkyle de formule

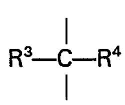

où $R^3$ et $R^4$ sont des groupes alkyles tels que définis dans la revendication 1 ou (2) un groupe cycloalkyle ou un groupe alcaryle tel que défini dans la revendication 1, $R^5$ est un radical alkyle, aryle, alkylidène, cycloalkyle ou alcaryle tel que défini dans la revendication 1 et $R^5$ et $R^6$ sont des hydrogènes, des groupes alkyles, hydroxyalkyles, aminoalkyles, cycloalkyles, aryle ou alcaryle tels que définis dans la revendication 1.

14. Amides substitués par un radical (3,5-dialkyl-4-hydroxyphényle) selon la revendication 13, dans lesquels $R^1$ est un groupe alkyle contenant 1 à 8 atomes de carbone, $R^2$ est un groupe alkyle contenant 1 à 5 atomes de carbone, $R^3$ et $R^4$ sont des groupes alkyles contenant 1 à 8 atomes de carbone, (1) est un groupe alkyle contenant 1 à 8 atomes de carbone et (2) est un groupe cyclohexyle, méthylcyclohexyle, cycloheptyle ou dicyclohexyle et, dans $R^5$, le groupe alkyle ou alkylidène contient 1 à 8 atomes de carbone, le groupe cycloalkyle contient 5 à 12 atomes de carbone et l'alcaryle est un radical phényle avec des groupes alkyles ayant 1 à 3 atomes de carbone et au moins un des $R^5$ et $R^6$ est un groupe alkyle, hydroxyalkyle, aminoalkyle, aryle, cycloalkyle ou alcaryle.

15. Amides substitués par un radical 2-(3,5-dialkyl-4-hydroxyphényle) selon la revendication 14, dans lesquels au moins un des $R^1$ et $R^2$ est un t-butyle ou un t-amyle, $R^3$ et $R^4$ sont des groupes alkyles contenant 1 à 4 atomes de carbone et chacun des $R^5$ et $R^6$ contient 1 à 8 atomes de carbone.

16. Un composé selon la revendication 15 choisi parmi
le N,N'-dibutyl-2-(3,5-di-t-butyl-4-hydroxyphényl)-2-méthylpropionate,
le N,N'-diéthyl-2-(3,5-di-t-butyl-4-hydroxyphényl)-2-méthylbutanamide,
le N,N'-diéthyl-2-(3,5-di-t-butyl-4-hydroxyphényl)-2,2-pentaméthylèneacétamide et
le N,N'-dibutyl-2-(3-t-butyl-5-méthyl-4-hydroxyphényl)-2-méthylpropionamide.